# FASCICULE DE BREVET EUROPEEN

(11) **EP 0 793 474 B1**
(45) Date de publication et mention de la délivrance du brevet: **07.07.1999**
(21) Numéro de dépôt: 95940340.3
(22) Date de dépôt: 21.11.1995
(51) Int. Cl.: A61K 6/087, A61K 6/027, A61L 27/00, C08K 9/02

(54) **ELEMENT PROTHETIQUE, ET NOTAMMENT TENON DENTAIRE EN MATERIAU COMPOSITE**
PROTHESENELEMENT, INSBESONDERE AUS EINEM VERBUNDWERKSTOFF HERGESTELLTER ZAHNSTIFT
PROSTHETIC ELEMENT, PARTICULARLY A TOOTH POST MADE OF COMPOSITE MATERIAL

(30) Priorité: 21.11.1994 FR 9413910
(43) Date de publication de la demande: 10.09.1997
(73) Titulaire: Reynaud, Marc, F-38240 Meylan (FR); Reynaud, Pierre-Luc, 38120 Saint-Egrève (FR)
(72) Inventeur: REYNAUD, Marc, F-38240 Meylan (FR); REYNAUD, Pierre-Luc, F-38120 Saint-Egrève (FR); CHU, Manh, F-38120 Saint-Egrève (FR)
(74) Mandataire: Bruder, Michel
(86) Numéro de dépôt international: FR9501532
(87) Numéro de publication internationale: WO9615759

(56) Documents cités:
- EP-A- 0 432 001
- EP-A- 0 462 512
- WO-A-89/04640
- DE-U- 9 400 070
- FR-A- 2 641 697
- GB-A- 2 028 855
- US-A- 3 493 403

## Description

La présente invention concerne un profilé en matériau composite destiné à constituer un élément prothétique, et notamment un tenon dentaire, ainsi qu'un procédé de fabrication d'un tel profilé.

On sait que, dans le domaine dentaire, afin d'assurer la pérennité de la substance dentaire d'une dent, on fait appel à des tenons métalliques qui sont vissés ou scellés dans celle-ci. De tels tenons métalliques présentent un certain nombre d'inconvénients, qui sont liés principalement à la grande différence qui existe entre les caractéristiques mécaniques de ces tenons et celles de la dentine dans laquelle ils sont disposés.

Afin d'éviter de tels inconvénients, on a proposé de faire appel à des tenons en matériau composite, constitués notamment d'une matrice de résine biocompatible, et notamment une résine époxy, dans laquelle sont noyées des fibres de carbone ou de verre.

Dans certains modes de mise en oeuvre, les fibres sont des fibres longitudinales qui s'étendent sur la totalité de la longueur du tenon et qui sont équitendues dans celui-ci. Les tenons ainsi constitués, en raison de leurs caractéristiques mécaniques proches de celles de la dentine, réduisent de façon notable les inconvénients précédemment mentionnés.

Cependant, de tels tenons présentent l'inconvénient d'être transparents à toute radiographie aux rayons X, si bien que leur positionnement est particulièrement difficile à établir à l'aide de l'appareillage classique dont disposent les praticiens.

Ces tenons sont habituellement fabriqués par pultrusion, c'est-à-dire par un procédé d'extrusion dans lequel on fait passer dans une même filière à la fois des fibres, et notamment des fibres de carbone ou de verre, et une matrice de résine, les fibres étant, tout au long de l'opération, maintenues sous tension. Cette opération de pultrusion est parfois difficile à mettre en oeuvre en raison des problèmes d'obturation de la filière de l'extrudeuse qui se manifestent en cours d'opération.

On connaît, dans l'état antérieur de la technique, des produits, ou charges, que l'on ajoute aux pâtes de reconstitution dentaire, afin de leur donner une certaine opacité aux rayons X. On a ainsi proposé dans ce but, dans le brevet américain US-A-4 503 169, d'inclure des oxydes de zirconium dans des pâtes de reconstitution dentaire.

On connaît également par le brevet GB-A-2.028855 une composition durcissable exempte de mercure à base d'un polyacide carboxylique et qui contient entre autre des éléments de remplissage qui peuvent éventuellement comprendre des éléments fibreux pouvant être revêtus d'oxydes métalliques susceptibles de réagir avec le polyacide carboxylique afin de favoriser la liaison avec la matrice.

La présente invention a pour but de proposer un moyen permettant de résoudre à la fois les problèmes liés à la mise en oeuvre de l'opération de pultrusion et ceux liés au défaut d'opacité des éléments prothétiques utilisés en art dentaire, qui sont constitués de matériaux composites à base de résines, notamment de résines époxy, renforcés par des fibres, notamment des fibres longues de carbone unidirectionnelles.

La présente invention a ainsi pour objet un profilé en matériau composite destiné à constituer un élément prothétique, et notamment un tenon dentaire, comportant une âme, constituée de fibres longitudinales, qui est noyée dans une matrice de résine, contenant au moins un oxyde métallique, caractérisé en ce que ledit oxyde métallique se présente sous la forme de granulés ou microbilles, dont les dimensions sont préférablement inférieures au diamètre moyen des fibres.

La demanderesse a en effet constaté que la mise en oeuvre de la pultrusion était grandement facilitée lorsque l'on ajoutait à la matrice de résine des oxydes métalliques. En effet l'oxyde métallique ajouté se comporte comme un agent de glissement qui réduit le collage de la résine et favorise l'écoulement du produit dans la filière de l'extrudeuse. La mise en oeuvre est encore plus facile lorsque les oxydes métalliques se présentent sous la forme de granulés ou de microbilles. Il est également possible, pour faciliter encore l'opération de pultrusion, d'utiliser des oxydes métalliques qui sont contenus dans des microbilles, et notamment des microbilles de verre.

Dans ces conditions les oxydes métalliques introduits dans la résine assurent deux fonctions, à savoir une première fonction de glissement favorisant l'extrusion et une seconde fonction d'opacité.

Ainsi qu'exposé ci-après, lorsque l'on est contraint d'ajouter des oxydes métalliques en quantité relativement importante dans la résine, on fait alors appel, en totalité ou en partie, à des oxydes contenus dans des microbilles de verre. Plusieurs particules d'oxydes peuvent d'ailleurs être groupées dans une même microbille.

Suivant l'invention, l'oxyde ou les oxydes métalliques choisis nécessaires pour conférer l'opacité souhaitée à l'élément prothétique peut être associé, en totalité ou en partie, aux fibres, c'est-à-dire qu'il est soit introduit dans celles-ci, soit disposé à leur surface de façon à constituer un revêtement en bonne adhérence avec celles-ci. Dans cette dernière forme de mise en oeuvre on améliore ainsi la cohésion des fibres avec la résine.

Dans un mode de mise en oeuvre de l'invention, l'oxyde métallique possède un indice de réfraction supérieur à l'indice de réfraction de la dentine. Un tel mode de mise en oeuvre de l'invention est particulièrement intéressant en ce qu'il permet, à opacité globale résultante identique, de diminuer la quantité dudit oxyde métallique contenu dans la résine, ce qui permet d'éviter d'amoindrir les qualités mécaniques du tenon. Il est ainsi possible de plus, pour un indice de réfraction souhaité donné, de contrôler la quantité d'oxyde métallique utilisé, afin de ne mettre dans le tenon que la quantité qui permet à l'agent glissant d'agir de façon optimale ce qui favorise l'opération de pultrusion.

La présente invention a également pour objet un procédé de fabrication d'un profilé en matériau composite, destiné à constituer un élément prothétique, et notamment un tenon dentaire, comportant une âme constituée de fibres longitudinales, cette âme étant noyée dans une matrice de résine, caractérisé en ce qu'il comporte les étapes consistant à mélanger à ladite matrice de résine au moins un oxyde métallique, et à extruder les fibres et la matrice de résine contenant ledit oxyde métallique, tout en maintenant lesdites fibres équitendues.

Dans un mode de mise en oeuvre de l'invention les oxydes métalliques peuvent être associés aux fibres, c'est à dire être introduits dans la masse de celles-ci ou à leur surface.

L'un au moins des oxydes métalliques associé aux fibres peut être identique à celui, ou à l'un de ceux, mélangé à la matrice de résine. Par ailleurs l'indice de réfraction de l'oxyde utilisé peut avantageusement être supérieur à l'indice de réfraction de la dentine.

Dans un mode de mise en oeuvre particulièrement intéressant de l'invention, les fibres enrobées d'oxydes métalliques reçoivent un agent de pontage spécifique destiné à favoriser leur liaison avec la matrice de résine, cet agent de pontage pouvant, dans la majorité des cas, être constitué de silanes.

Le revêtement d'oxyde métallique peut être réalisé sur toute la surface des fibres, notamment par une imprégnation thermique, lorsque la température de fusion des fibres est plus élevée que celle des oxydes métalliques. Dans de telles conditions, la granulométrie des oxydes métalliques n'a que peu d'influence sur la radio-opacité. On peut également réaliser ledit revêtement, notamment dans le cas où la température de fusion des fibres est inférieure à celle de l'oxyde métallique, en faisant appel à un procédé de projection et notamment un procédé de projection plasma.

Suivant ce mode de mise en oeuvre, les fibres provenant de bobines de stockage traversent un bac d'imprégnation où elles sont imprégnées d'oxyde métallique à l'état fondu, puis ces fibres sont essorées et séchées dans une seconde enceinte dont la température diminue progressivement.

On décrira ci-après, à titre d'exemple non limitatif, une forme d'exécution de la présente invention, en référence au dessin annexé sur lequel :
- La figure 1 est une vue schématique illustrant un mode de mise en oeuvre général de profilés suivant l'invention.
La figure 2 est une vue schématique d'une variante de mise en oeuvre de l'invention.

Suivant l'invention on constitue des tenons dentaires en incorporant à une matrice constituée d'une résine thermodurcissable, ou d'une résine thermoplastique, des oxydes métalliques qui sont biocompatibles, afin de ne pas provoquer de problèmes d'acceptation de la part de l'organisme du patient. La quantité d'oxyde métallique introduite dans la résine est fonction de l'indice de réfraction que l'on souhaite obtenir. De préférence ces oxydes possèdent un indice de réfraction supérieur à celui de la dentine, voire à celui de la structure osseuse et, de préférence, très supérieur à celles-ci.

On sait que l'indice de réfraction de la structure osseuse est de l'ordre de 1,65 et que celle de la dentine est, quant à elle, de l'ordre de 1,6. Dans ces conditions, pour que l'élément prothétique dentaire soit détectable par radiographie aux rayons X il devra posséder un indice de réfraction différent de celui de la dentine et/ou de la structure osseuse, soit qu'il soit légèrement supérieur ou inférieur à ceux-ci. Les indices de réfraction du tenon dentaire devront ainsi être soit supérieurs à 1,65 soit inférieurs à 1,6 si l'on veut pouvoir les distinguer à la fois de la dentine ou de la structure osseuse.

Les oxydes métalliques les plus intéressants, qui sont ainsi susceptibles d'être utilisés dans la mise en oeuvre de la présente invention sont :
- l'oxyde de magnésium: MgO n = 1,74
- l'oxyde de strontium: SrO n = 1,81
- l'oxyde de calcium: CaO₂ n = 1,89
- l'oxyde de baryum: BaO n = 1,98 BaO₂ n = 1,98
- l'oxyde de zinc: ZnO n = 2,01 à 2,03
- l'oxyde de zirconium: ZrO₂ n = 2,13 à 2,20
- l'oxyde de titane: TiO₂ n = 2,61 à 2,90

La quantité d'oxyde métallique introduite dans la résine dépend de l'indice de réfraction que l'on souhaite donner au tenon.

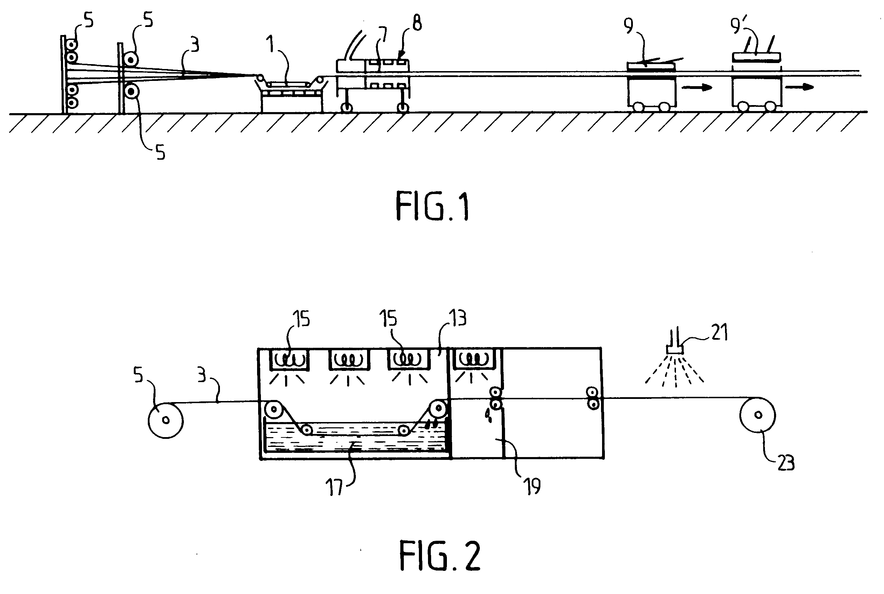

Dans une première étape du procédé suivant l'invention, on mélange tout d'abord à une matrice de résine l'oxyde métallique choisi, par exemple par malaxage. Dans une seconde étape, comme représenté sur la figure 1, on fait passer dans un bac 1, contenant le mélange de résine et d'oxydes métalliques choisis, un faisceau de fibres 3, notamment des fibres de carbone ou de verre, qui sont stockées sur des bobines 5. L'ensemble traverse une filière 7 d'une extrudeuse 8 et, simultanément à cette extrusion, les fibres 3 sont soumises à une tension par un système de tirage constitué, de façon connue, par des "chenilles" 9, 9'. En sortie de la filière 7, le profilé composite obtenu est polymérisé. Si un tel profilé est destiné à constituer des tenons dentaires, il est ensuite découpé à la longueur souhaitée et éventuellement usiné.

Dans un premier exemple de mise en oeuvre de l'invention on réalise un mélange de résine époxy avec 25% en poids d'oxyde de baryum et 25% en poids d'oxyde de titane. Physiquement les oxydes métalliques introduits dans la matrice de résine se présentent sous la forme de granulés dont la granulométrie moyenne est inférieure au diamètre moyen des fibres. Les fibres utilisées sont des fibres de carbone haute performance d'un diamètre de l'ordre de 8 µm qui sont préférablement rassemblées en mèches de 3.000 à 6.000 filaments. Le diamètre de la filière 7 est tel que l'on obtient, en sortie de l'extrudeuse 8, un profilé d'un diamètre d'environ 2 mm. Un tel profilé est particulièrement apte à être utilisé pour réaliser des tenons dentaires.

La radio-opacité du profilé obtenu s'est révélée supérieure à celle de la dentine, son indice de réfraction étant de 1,69. Un tel profilé, s'il est utilisé comme tenon dentaire, est parfaitement localisable dans les examens en radiographie X, lorsque l'on observe la dent du côté de la racine.

Dans un second exemple de mise en oeuvre de l'invention, on réalise un mélange de résine époxy avec 39 % en poids d'oxyde de titane. Physiquement l'oxyde de titane mélangé à la résine se présente, comme précédemment, sous la forme de microbilles dont la granulométrie moyenne est de l'ordre de 2 µm Les fibres utilisées sont des fibres de carbone haute performance d'un diamètre de l'ordre de 8 µm. Le diamètre de la filière utilisée est le même que précédemment.

A l'examen, la radio-opacité du profilé obtenu s'est révélée être très supérieure, non seulement à celle de la dentine, mais également à celle de la structure osseuse, et à celle de l'émail de la dent (n = 1,65), son indice de réfraction étant de 1,82, si bien qu'un tel profilé est localisable par radiographie X même à travers l'émail d'une dent.

Comme mentionné précédemment, on a constaté lors des différentes mises en oeuvre de l'invention, que le passage du produit (c'est à dire de l'ensemble constitué d'une part du mélange de résine et d'oxydes métalliques et d'autre part des fibres) dans la filière de l'extrudeuse était rendu plus difficile, lorsque les profilés étaient de faible diamètre et qu'ils nécessitaient, pour atteindre l'indice de réfraction souhaité, des quantités importantes d'oxydes métalliques.

Afin d'éviter cet inconvénient, on enrobe les fibres elles-mêmes d'un oxyde métallique donné, ou d'un mélange de plusieurs oxydes métalliques. Dans un tel mode de mise en oeuvre, les fibres peuvent recevoir éventuellement un traitement préalable destiné à favoriser leur accrochage avec les oxydes métalliques dont on souhaite les enrober. Après un tel enrobage les fibres peuvent être stockées en bobine en attendant leur utilisation ultérieure. Une telle façon de procéder permet, pour une quantité déterminée d'oxydes métalliques nécessaires pour assurer l'opacité souhaitée, de diminuer celle mélangée à la matrice de résine, ce qui diminue la compacité du mélange résine/oxyde métallique et améliore du même coup le passage du produit dans la filière.

Il est ainsi possible d'obtenir des profilés en matériau composite à fibres de carbone équitendues qui possèdent des indices de réfraction élevés, très supérieurs à ceux de la structure osseuse voisine, ce qui présente un grand intérêt dans nombre d'applications médicales telles que par exemple les implants articulaires et....

Pour enrober les fibres avec les oxydes métalliques on les fait passer de préférence dans un bain contenant les oxydes métalliques en fusion. Lorsque la température de fusion d'un oxyde donné est supérieure à celle de la fibre, ce qui est notamment le cas lorsque l'on souhaite revêtir des fibres de verre avec de l'oxyde de titane, on peut avoir recours à un procédé de projection sous plasma de l'oxyde métallique.

Dans un troisième exemple de mise en oeuvre de l'invention on reprend les proportions d'oxyde de titane et d'oxyde de baryum données au premier exemple, mais en déposant l'oxyde de baryum sur les fibres et en mélangeant l'oxyde de titane à la résine. De cette façon les fibres peuvent être revêtues par immersion à chaud car l'on n'est pas gêné par la valeur élevée de la température de fusion de l'oxyde de titane, puisque celui-ci est mélangé à la résine. Il est ainsi possible, dans ce cas, d'utiliser non seulement des fibres de carbone mais également des fibres de verre puisque la température de fusion de l'oxyde de baryum est inférieure à celle du verre.

On peut également, suivant l'invention, mélanger à la matrice de résine le même oxyde métallique que celui déposé sur les fibres.

On peut bien entendu mélanger à la résine plus de deux oxydes métalliques. On décrira ainsi ci-après un quatrième exemple de mise en oeuvre de l'invention dans lequel on a mélangé à une résine époxy 8,5 % en poids d'oxyde de zirconium, 10,5 % en poids d'oxyde de titane et 17,5 % en poids d'oxyde de baryum.

Les oxydes métalliques de titane et de baryum sont, dans le présent mode de mise en oeuvre de l'invention, introduits sous la forme de microbilles. Ces oxydes sont l'un et l'autre inclus dans une même microbille de verre de granulométrie de l'ordre de 20 à 40 µm. L'oxyde de zirconium se présente sous la forme de granulés dont la granulométrie est de l'ordre du micromètre. Les fibres utilisées sont des fibres de carbone haute performance d'un diamètre de l'ordre de 8 µm. Le diamètre de la filière est tel que l'on obtienne en sortie de l'extrudeuse un profilé d'un diamètre de 2 mm.

A l'examen, la radio-opacité du profilé obtenu s'est révélée légèrement supérieure à celle de la dentine puisque son indice de réfraction est de 1,66. Une simple radiographie dentaire d'un profilé inséré dans une dent a confirmé une radio-opacité de ce profilé légèrement supérieure à celle de la dentine.

Les oxydes métalliques disposés dans des microbilles se sont révélés des agents glissants particulièrement efficaces, ce qui permet de les introduire en grande quantité dans la résine sans provoquer de bourrage au niveau de la filière de l'extrudeuse. Leur utilisation permet, pour un indice de réfraction souhaité donné, de contrôler la quantité d'oxyde métallique utilisé, afin de ne mettre dans le tenon que la quantité qui permet à l'agent glissant d'agir de façon optimale ce qui favorise l'opération de pultrusion.

Afin d'améliorer l'adhérence des fibres, ou des fibres enrobées par des oxydes, avec la matrice de résine on peut réaliser leur ensimage préalablement à leur introduction dans la résine. Un tel ensimage consiste, de façon connue, à réaliser un traitement de surface des fibres, enrobées ou non, à l'aide d'un élément "liant" dont le rôle est de constituer un pontage chimique avec les molécules de la matrice de résine.

Pour ce faire, ainsi que représenté sur la figure 2 on admet des fibres 3 stockées sur des bobines 5, dans une enceinte 13 chauffée par des résistances 15, où les oxydes métalliques 17 sont maintenus à l'état fondu, puis dans une enceinte d'essorage et séchage 19 où la température diminue progressivement. En sortie de l'enceinte 19 des moyens de pulvérisation 21 projettent sur les fibres l'élément liant choisi, et finalement les fibres traitées sont enroulées sur une bobine de stockage 23.

Les oxydes métalliques peuvent également être incorporés à la fibre elle-même. Ainsi, notamment dans le cas de fibres de verre, les oxydes métalliques peuvent être introduits dans les fibres lors de leur fabrication.

Les oxydes métalliques peuvent se présenter à la fois, dans un même profilé, sous la forme de granulés, ou microbilles, et sous une forme hétérogène. Préférentiellement, ils se présenteront en majorité sous forme de microbilles.

## Revendications

1. Profilé en matériau composite destiné à constituer un élément prothétique, et notamment un tenon dentaire, comportant une âme, constituée de fibres longitudinales, qui est noyée dans une matrice de résine, contenant au moins un oxyde métallique, caractérisé en ce que ledit oxyde métallique se présente sous la forme de granulés ou microbilles, dont les dimensions sont préférablement inférieures au diamètre moyen des fibres.

2. Profilé suivant la revendication 1 caractérisé en ce qu'au moins un oxyde métallique est associé aux fibres.

3. Profilé suivant la revendication 2 caractérisé en ce que le susdit oxyde métallique associé aux fibres est disposé à la surface de celles-ci.

4. Profilé suivant l'une des revendications précédentes, caractérisé en ce que ledit oxyde métallique possède un indice de réfraction supérieur à l'indice de réfraction de la dentine.

5. Profilé suivant la revendication 2 caractérisé en ce que les fibres sont des fibres de verre et les susdits oxydes métalliques associés aux fibres sont incorporés à l'intérieur de celles-ci.

6. Profilé suivant l'une des revendications 2 à 5 caractérisé en ce que ledit oxyde métallique est commun avec celui ou l'un de ceux contenus dans la matrice de résine.

7. Profilé suivant l'une des revendications précédentes caractérisé en ce que ledit oxyde métallique est contenu dans une microbille de verre.

8. Profilé suivant l'une quelconque des revendications précédentes caractérisé en ce que les fibres sont revêtues d'un agent de pontage destiné à les relier à la matrice de résine.

9. Procédé de fabrication d'un profilé en matériau composite, destiné à constituer un élément prothétique, et notamment un tenon dentaire, comportant une âme constituée de fibres longitudinales, cette âme étant noyée dans une matrice de résine, caractérisé en ce qu'il comporte les étapes consistant à mélanger à ladite matrice de résine au moins un oxyde métallique, et à extruder les fibres et la matrice de résine contenant ledit oxyde métallique.

10. Procédé suivant la revendication 9 caractérisé en ce qu'il comprend l'étape consistant à associer aux fibres au moins un oxyde métallique, dont l'indice de réfraction est supérieur à l'indice de réfraction de la dentine.

11. Procédé suivant la revendication 10 caractérisé en ce que l'on dispose le susdit oxyde métallique associé aux fibres à la surface de celles-ci, à la façon d'un revêtement.

12. Procédé suivant la revendication 11 caractérisé en ce que le revêtement d'oxyde métallique est réalisé, sur toute la surface des fibres par un passage dans un bain contenant au moins un oxyde métallique fondu.

13. Procédé suivant la revendication 11 caractérisé en ce que le revêtement d'oxyde métallique est réalisé par un procédé de projection plasma.

14. Procédé suivant l'une des revendications 9 à 13 caractérisé en ce que les fibres sont des fibres de verre et le susdit oxyde métallique associé aux fibres est incorporé à celles-ci au cours de leur fabrication.

15. Procédé suivant l'une des revendications 9 à 14 caractérisé en ce que l'un au moins des oxydes métalliques associé aux fibres est identique à celui, ou l'un de ceux, mélangé à la matrice de résine.

16. Procédé suivant la revendication 10 caractérisé en ce que les fibres traversent un bac d'imprégnation (13) où elles sont imprégnées d'au moins un oxyde métallique à l'état fondu, puis ces fibres sont essorées et séchées dans une enceinte (19) dont la température diminue progressivement.

17. Procédé suivant l'une des revendications 9 à 16 caractérisé en ce que les fibres, après avoir reçu ledit oxyde métallique associé, sont recouvertes d'un agent de pontage spécifique destiné à les relier à la matrice de résine.

18. Procédé suivant la revendication 17 caractérisé en ce que l'agent de pontage est un silane.

## Claims

1. Shaped member made of composite material intended for making a prosthetic element, particularly a tooth post, comprising a core constituted by longitudinal fibers, which is embedded in a resin matrix, containing at least one metal oxide, characterized in that said metal oxide is in the form of granules or microballs, whose dimensions are preferably smaller than the mean diameter of the fibers.

2. Shaped member according to Claim 1, characterized in that at least one metal oxide is associated with the fibers.

3. Shaped member according to Claim 2, characterized in that the said metal oxide associated with the fibers is disposed on the surface of the latter.

4. Shaped member according to one of the preceding Claims, characterized in that said metal oxide presents a refractive index greater than the refractive index of dentine.

5. Shaped member according to Claim 2, characterized in that the fibers are glass fibers and the said metal oxides associated with the fibers are incorporated within said fibers.

6. Shaped member according to one of Claims 2 to 5, characterized in that said metal oxide is common with that or one of those contained in the resin matrix.

7. Shaped member according to one of the preceding Claims, characterized in that said metal oxide is contained in a glass microball.

8. Shaped member according to any one of the preceding Claims, characterized in that the fibers are coated with a coupling agent intended to connect them to the resin matrix.

9. Process for manufacturing a shaped member of composite material, intended for constituting a prosthetic element, particularly a tooth post, comprising a core constituted by longitudinal fibers, this core being embedded in a resin matrix, characterized in that it comprises the steps consisting in mixing with said resin matrix at least one metal oxide, and in extending the fibers and the resin matrix containing said metal oxide.

10. Process according to Claim 9, characterized in that it comprises the step consisting in associating with the fibers at least one metal oxide, whose refractive index is greater than the refractive index of dentine.

11. Process according to Claim 10, characterized in that said metal oxide associated with the fibers is disposed on the surface of said fibers, in the manner of a coating.

12. Process according to Claim 11, characterized in that the coating of metal oxide is effected, on the whole surface of the fibers, by a passage in a bath containing at least one molten metal oxide.

13. Process according to Claim 11, characterized in that the metal oxide coating is effected by a plasma projection process.

14. Process according to one of Claims 9 to 13, characterized in that the fibers are glass fibers and said metal oxide associated with the fibers is incorporated in said fibers while they are being manufactured.

15. Process according to one of Claims 9 to 14, characterized in that at least one of the metal oxides associated with the fibers is identical to that, or one of those, mixed with the resin matrix.

16. Process according to Claim 10, characterized in that the fibers pass through an impregnation tank (13) where they are impregnated with at least one metal oxide in the molten state, then these fibers are dewatered and dried in an enclosure (19) of which the temperature decreases progressively.

17. Process according to one of Claims 9 to 16, characterized in that the fibers, after having received said associated metal oxide, are coated with a specific coupling agent intended to connect them to the resin matrix.

18. Process according to Claim 17, characterized in that the coupling agent is a silane.

## Patentansprüche

1. Profil aus Verbundwerkstoff, dazu bestimmt, ein prothetisches Element und insbesondere einen Zahnstift zu bilden, aufweisend einen Kern, gebildet aus Längsfasern, der in eine Harzmatrize versenkt ist, die mindestens ein Metalloxid enthält,
dadurch gekennzeichnet, daß das Metalloxid in Form von Granülen oder Mikrokügelchen dargeboten ist, deren Abmessungen vorzugsweise kleiner als der mittlere Durchmesser der Fasern sind.

2. Profil nach Anspruch 1,
dadurch gekennzeichnet, daß mindestens ein Metalloxid mit den Fasern verbunden ist.

3. Profil nach Anspruch 2,
dadurch gekennzeichnet, daß das vorgenannte, mit den Fasern verbundene Metalloxid auf der Oberfläche der Fasern angeordnet ist.

4. Profil nach einem der vorhergehenden Ansprüche,
dadurch gekennzeichnet, daß das Metalloxid einen größeren Brechungskoeffizient besitzt als der Brechungskoeffizient des Dentins.

5. Profil nach Anspruch 2,
dadurch gekennzeichnet, daß die Fasern Glasfasern sind und die vorgenannten, mit den Fasern verbundenen Metalloxide in das Innere der Fasern eingebettet sind.

6. Profil nach einem der Ansprüche 2 bis 5,
dadurch gekennzeichnet, daß das Metalloxid mit dem oder einem derjenigen gemein ist, die in der Harzmatrize enthalten sind.

7. Profil nach einem der vorhergehenden Ansprüche,
dadurch gekennzeichnet, daß das Metalloxid in einem Glasmikrokügelchen enthalten ist.

8. Profil nach irgendeinem der vorhergehenden Ansprüche, dadurch gekennzeichnet, daß die Fasern mit einem Haftvermittler überzogen sind, der zu bestimmt ist, sie mit der Harzmatrize zu verbinden.

9. Verfahren zur Herstellung eines Profils aus Verbundwerkstoff, welches Profil dazu bestimmt ist, ein prothetisches Element und insbesondere einen Zahnstift zu bilden, aufweisend einen Kern, der aus Längsfasern gebildet ist, wobei dieser Kern in eine Harzmatrize versenkt ist, dadurch gekennzeichnet, daß das Verfahren die Schritte aufweist, die darin bestehen, die Harzmatrize mit mindestens einem Metalloxid zu vermischen und die Fasern und die das Metalloxid enthaltende Harzmatrize zu extrudieren.

10. Verfahren nach Anspruch 9,
dadurch gekennzeichnet, daß es den Schritt umfaßt, der darin besteht, die Fasern mit mindestens einem Metalloxid zu verbinden, dessen Brechungskoeffizient größer als der Brechungskoeffizient des Dentins ist.

11. Verfahren nach Anspruch 10,
dadurch gekennzeichnet, daß das vorgenannte, mit den Fasern verbundene Metalloxid an der Oberfläche der Fasern in der Art eines Überzugs angeordnet wird.

12. Verfahren nach Anspruch 11,
dadurch gekennzeichnet, daß der Metalloxidüberzug auf der gesamten Oberfläche der Fasern durch ein Durchlaufen eines Bads ausgeführt wird, welches mindestens ein geschmolzenes Metalloxid enthält.

13. Verfahren nach Anspruch 11,
dadurch gekennzeichnet, daß der Metalloxidüberzug durch ein Plasmaprojektionsverfahren ausgeführt wird.

14. Verfahren nach einem der Ansprüche 9 bis 13,
dadurch gekennzeichnet, daß die Fasern Glasfasern sind und das vorgenannte, mit den Fasern verbundene Metalloxid in die Glasfasern während ihrer Herstellung eingebettet wird.

15. Verfahren nach einem der Ansprüche 9 bis 14,
dadurch gekennzeichnet, daß mindestens eines der Metalloxide, die mit den Fasern verbunden sind, mit demjenigen oder einem derjenigen identisch ist, das mit der Harzmatrize vermischt ist.

16. Verfahren nach Anspruch 10,
dadurch gekennzeichnet, daß die Fasern ein Imprägnierbad (13) durchqueren, wo sie mit mindestens einem, in geschmolzenem Zustand befindlichen Metalloxid imprägniert werden, und diese Fasern daraufhin luftgetrocknet bzw. trockengeschleudert und in einem Raum (19) ausgetrocknet werden, dessen Temperatur schrittweise abnimmt.

17. Verfahren nach einem der Ansprüche 9 bis 16,
dadurch gekennzeichnet, daß die Fasern, nachdem sie das verbundene Metalloxid empfangen haben, mit einem spezifischen Haftvermittler bedeckt werden, der dazu bestimmt ist, sie mit der Harzmatrize zu verbinden.

18. Verfahren nach Anspruch 17,
dadurch gekennzeichnet, daß der Haftvermittler ein Silan ist.
